(19) Europäisches Patentamt
European Patent Office
Office européen des brevets

(11) Veröffentlichungsnummer : **0 362 638 B1**

# (12) EUROPÄISCHE PATENTSCHRIFT

(45) Veröffentlichungstag der Patentschrift : **02.12.92 Patentblatt 92/49**

(51) Int. Cl.[5] : **C07C 49/84,** C07C 45/64

(21) Anmeldenummer : **89117506.9**

(22) Anmeldetag : **22.09.89**

## (54) Verfahren zur Herstellung von symmetrischen und unsymmetrischen Monoacetalen aromatischer 1,2-Diketone.

(30) Priorität : **06.10.88 DE 3834029**

(43) Veröffentlichungstag der Anmeldung : **11.04.90 Patentblatt 90/15**

(45) Bekanntmachung des Hinweises auf die Patenterteilung : **02.12.92 Patentblatt 92/49**

(84) Benannte Vertragsstaaten : **AT BE CH DE ES FR GB IT LI NL SE**

(56) Entgegenhaltungen : **DE-A- 2 365 852**

(56) Entgegenhaltungen : **PATENT ABSTRACTS OF JAPAN, Band 6, Nr. 3 (C-86)(881), 9. Januar 1982; & JP-A-56 128 729 CHEMISCHE BERICHTE, Band 94, 1961, Seiten 2258-2263, Weinheim-Bergstrasse, D; R. KUHN et al: "Monoketale von 1.2-Diketonen",Seite 2260, Zeilen 23-42; Seite 2261, Zeilen 1-4**

(73) Patentinhaber : **BASF Aktiengesellschaft
Carl-Bosch-Strasse 38
W-6700 Ludwigshafen (DE)**

(72) Erfinder : **Boettcher, Andreas, Dr.
Konrad-Adenauer-Ring 38
W-6907 Nussloch (DE)**

EP 0 362 638 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein neuartiges Verfahren zur Herstellung von symmetrischen und unsymmetrischen Monoacetalen aromatischer 1,2-Diketone in einem unpolaren Lösungsmittel in Gegenwart von Harnstoffderivaten als Katalysatoren.

Verbindungen vom Typ der cyclischen und nichtcyclischen, symmetrischen Benzil-1,1-acetale sind bekannte Photoinitiatoren und werden beispielsweise in den DE-A 22 32 365, DE-A 23 37 813 und der EP 85 031 beschrieben. Derivate des 9,9′-Phenanthrenchinondimethylacetals bzw. des Acenaphthenchinons haben pharmakologische Wirksamkeit.

Zur Herstellung der Monoacetale des Benzils und des Phenanthrenchinons verwenden Kuhn et al. (Chem. Ber. 94, 2258 (1961)) Bariumoxid (Bariumhydroxid) und Methyljodid in Dimethylformamid. Von den gleichen Autoren wird wasserfreies Ninhydrin in Methanol in Gegenwart 5 gew.%iger methanolischer Salzsäure mit Dimethylsulfit in das stabile 1,1-Dimethylacetal überführt. Dieses Syntheseprinzip wurde auf Acetale des Benzils übertragen. Dabei setzt man das aromatische 1,2-Diketon mit einem Schwefeligsäureester in Gegenwart einer wasserfreien Säure in einem primären Monoalkohol als Lösungsmittel oder mit in situ aus Thionylchlorid in überschüssigem Alkohol erzeugtem Schwefligsäureester um, was beispielsweise in den DE-A 23 37 813, DE-A 23 65 497, DE-A 23 65 852, AT 331 237 und ES 508 476 beschrieben ist.

In anderen Veröffentlichungen werden Orthoameisensäuretrialkylester als Alkylierungsmittel vorgeschlagen. So wird Benzil mit Orthoameisensäuretrimethylester in überschüssigem Methanol in Gegenwart von Schwefelsäure in das entsprechende Dimethylketal überführt (Chem. Abstr. 96, 34871, JP 56 128-729). Nach K.C. Rice (Synthesis 1988, 233) werden in Nitromethan mit Trifluormethansulfonsäure und dem Orthoester bessere Ergebnisse erzielt.

Acenaphtenchinon kann mit p-Toluolsulfonsäure, Zinkchlorid, Zinkchlorid/konz. Schwefelsäure oder Eisen(III)sulfat/konz. Schwefelsäure in absolutem Alkohol in das entsprechende Ketal überführt werden (J. Tsunetsugu, J. Chem. Soc. Perkin Trans.I 1986, 1965).

Einen anderen Weg gehen G. LiBassi et al. (EP 85 031): Benzoine werden in Dimethylformamid mit Sulfurylchlorid zum entsprechenden Dichlorid umgesetzt, aus dem mit Natriumalkoholat in Alkohol das symmetrische Acetal gebildet wird.

Allen bisher genannten Verfahren ist gemeinsam, daß

1. ausschließlich symmetrische Acetale
2. mit sauren Katalysatoren hergestellt werden.

Es ist nun literaturbekannt, daß Acetale und Ketale in Gegenwart saurer Katalysatoren in guten bis sehr guten Ausbeuten in die entsprechenden Carbonylverbindungen und Alkohole zurückgespalten werden können (vgl. beispielsweise Houben-Weyl, VII/1, 423; VII/2a, 790; Organic Synth. Coll. Vol. III 217, 731 (1955)). Die Darstellung der Ketale gelingt daher nur in akzeptablen Ausbeuten, wenn das Alkylierungsreagenz im Überschuß vorhanden ist und bei der Aufarbeitung keine Säure freigesetzt wird. Weiterhin sind alle bisher genannten Verfahren mit dem Nachteil behaftet, daß sie bei der Übertragung in einem größeren Maßstab schlecht reproduzierbare Ausbeuten liefern, hohe Temperaturen und lange Reaktionszeiten erfordern.

Diese Probleme werden umgangen, wenn die Reaktion von vornherein im alkalischen Milieu durchgeführt wird. Aromatische Benzile können so in hoher Ausbeute in Gegenwart von Alkalialkoholaten mit Arylsulfonsäureestern oder Alkylsulfaten in die 1,1-Acetale überführt werden, was aus den DE-A 26 16 382, DE-A 26 16 408 und DE-A 26 16 588 bekannt ist. Dieses Verfahren erlaubt insbesondere auch die Darstellung unsymmetrischer Ketale, die auf anderem Wege nicht zugänglich sind. Bei den bisher beschriebenen Verfahren zur Darstellung von Ketalen aromatischer 1,2-Diketone ist die Eigenschaft des verwendeten Lösungsmittels besonders wichtig; in allen Fällen werden ausschließlich polare Lösungsmittel, wie z.B. Dioxan, Dimethylformamid oder Methanol bevorzugt, weil sie nicht nur für die nucleophile Substitution bzw. die Alkylierungsreaktion optimal sind, sondern auch bei der Aufarbeitung der Reaktionsansätze durch Mischen mit Wasser bequem entfernt werden können. Diese Arbeitsweise ist aber aus der Sicht der Abwasserbelastung unbefriedigend und es müssen sich in der Regel kostenintensive Reinigungsverfahren anschließen, um die Lösungsmittel wieder aus dem Abwasser zu entfernen. Es ist allgemein bekannt, daß ein mit Wasser mischbares organisches Lösungsmittel nur mit aufwendigen Methoden, wie z.B. Destillation oder Extraktion aus der wäßrigen Phase entfernt werden kann. Häufig werden dazu Azeotrope mit Wasser gebildet, die eine vollständige Rückgewinnung unmöglich machen.

Ein anderer Aspekt der Lösungsmittelproblematik wird am System Thionylchlorid/Methanol offenkundig. Das Alkylierungsmittel wird dabei bei der Reaktion in überschüssigem Methanol erzeugt. Es entstehen die aggressiven sauren Gase $SO_2$ und HCl; außerdem kann die Bildung von Methylchlorid nicht ausgeschlossen werden.

Bei dem in den DE-PS 26 16 382, 26 16 408 und 26 16 588 beschriebenen Verfahren ist die Bildung aggressiver Gase ausgeschlossen. Es wird die Verwendung von Dioxan und Dimethylformamid bevorzugt. Das letztere Lösungsmittel reagiert allerdings mit dem Natriummethylat/Dimethylsulfat bei höheren Temperaturen unter Bildung von Dimethylformamid-dimethylacetal. Das Alkylierungsmittel wird somit für diese Reaktion verbraucht.

In der DE-C 26 16 382 werden auch aromatische Lösungsmittel wie Benzol, Toluol, Xylol oder o-Dichlorbenzol als geeignet genannt. Im Falle des aus dem Benzil zugänglichen Benzildimethylketals beträgt die Gesamtausbeute in Toluol als Lösungsmittel aber weniger als 50 %. Benzol, Xylol oder o-Dichlorbenzol verhalten sich ähnlich.

Auch der Zusatz von Lösungsvermittlern für Alkalialkoholate, wie z.B. Ethylenglykoldimethylether, Diethylenglykolmonobutylether, Polyethylenglykoldimethylether oder Pluriol® E 1000 (Polyethylenglykol der BASF) ergibt eine schlechte BDK-Ausbeute und die Bildung von Dimethylether. Dies ist darauf zurückzuführen, daß in Gegenwart der genannten Lösungsvermittler in Toluol die Alkylierung von Natriummethylat durch Dimethylsulfat bevorzugt wird. Bei Verwendung von polaren Lösungsmitteln läuft die Reaktion in die gewünschte Richtung.

Der Erfindung lag die Aufgabe zugrunde, ein umweltfreundliches Verfahren zur Herstellung von symmetrischen Monoacetalen aromatischer 1,2-Diketone in einem rückgewinnbaren unpolaren organischen Lösungsmittel zu finden, das zudem noch hohe Ausbeuten liefern sollte.

Die Aufgabe wurde durch Mitverwendung von speziellen Harnstoffderivaten als Katalysatoren bei der Alkylierungsreaktion in unpolaren organischen Lösungsmitteln gelöst. Dadurch werden nicht nur die Ausbeuten erhöht, sondern auch die Nebenreaktionen unterdrückt und die Reaktionszeiten verkürzt.

Gegenstand der vorliegenden Erfindung ist ein Verfahren zur Herstellung von symmetrischen und unsymmetrischen 1,1-Acetalen aromatischer 1,2-Diketone der allgemeinen Formel I

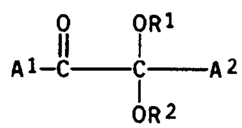

I

worin

$A^1$ und $A^2$ für gleiche, verschiedene oder miteinander verbundene, gegebenenfalls geradkettige oder verzweigte Alkyl- oder Alkoxy-Reste mit 1 bis 6 C-Atomen, Halogen- oder Phenylreste tragende, aromatische Reste stehen und

$R^1$ und $R^2$ für gleiche oder verschiedene, gegebenenfalls substituierte, geradkettige oder verzweigte Alkylreste mit 1 bis 10 C-Atomen stehen,

aus den entsprechenden aromatischen 1,2-Diketonen der allgemeinen Formel II

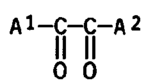

II

dadurch gekennzeichnet, daß man die 1,2-Diketone in einem unpolaren aprotischen organischen Lösungsmittel mit einem Alkylierungsmittel

$$(R^1)_nX$$

und einem Alkoholat

$$(R^2O)_mM,$$

wobei n und m für ganze Zahlen von 1 bis 3 stehen,

X für den Rest einer ein- bis dreibasigen Säure und

M für ein Metall der 1. bis 3. Hauptgruppe des Periodensystems der Elemente steht,

in Gegenwart eines Harnstoffderivates der allgemeinen Formel III oder IV

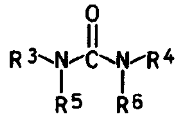

III

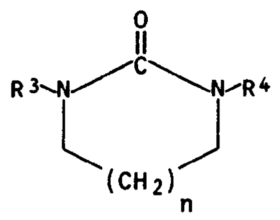

IV

oder eines Gemisches von III oder IV,
worin
n = 0 bis 3 und
$R^3$, $R^4$, $R^5$ und $R^6$ für gleiche oder verschiedene, geradkettige oder verzweigte Alkylreste mit 1 bis 6 C-Atomen oder Wasserstoff stehen als Katalysator bei Temperaturen von -20 bis +100°C umsetzt.

Zu den Einsatzstoffen ist folgendes auszuführen:

Die in diesem Verfahren verwendeten aromatischen Diketone sind bekannt und werden beispielsweise nach dem in der DE-A 36 24 146 beschriebenen Verfahren durch Oxidation der entsprechenden Benzoine hergestellt.

Geeignete aromatische Diketone der allgemeinen Formel II sind besonders solche, in denen die gegebenenfalls miteinander verbundenen Arylreste unabhängig voneinander je einen unsubstituierten oder durch Alkyl- oder Alkoxy mit 1 bis 6 C-Atomen, Halogen oder durch Phenylreste höchstens trisubstituierten Phenylrest bedeuten. Beispiele für aromatische 1,2-Diketone, die für dieses Verfahren verwendet werden können, sind Benzil und substituierte Benzile wie z.B. 4,4′-Dimethylbenzil, 4,4′-Diisopropylbenzil, 4,4′-Diphenylbenzil, 2,2′-Dimethoxybenzil, 4-Methylbenzil, 3-Methoxybenzil, 2,2′-Dimethylbenzil, 4-Chlor-4′-phenylbenzil, 4,4′-Dichlorbenzil, 3,3′-Dibrombenzil, 2,4,2′,4′-Tetramethylbenzil, 2,4,6-Trimethylbenzil, 2,4-Dichlor-4′-methylbenzil. Beispiele für aromatische 1,2-Diketone, bei denen die Arylreste miteinander verbunden sind und die für dieses Verfahren verwendet werden können, sind gegebenenfalls substituiertes Phenanthrenchinon, Acenaphthenchinon oder 1,2-Aceanthrenchinon. Diese o-Chinone sind nach literaturbekannten Verfahren und nach dem in J. Chem. Soc., Perkin Trans.I 1986, 1965 beschriebenen Verfahren darstellbar.

Für das Verfahren geeignete Alkylierungsmittel haben die genannte Formel $(R^1)_nX$ und sind Ester ein bis dreibasiger Säuren, insbesondere von ein Schwefel-, ein Phosphor- oder ein Halogenatom enthaltenden Säuren. Genannt seien die Ester der Schwefelsäure, der schwefligen Säure, der Phosphorsäure und der phosphorigen Säure, die Ester der Halogenwasserstoffsäure, wie die Chloride, Bromide und Jodide sowie der aliphatischen und aromatischen Sulfonsäuren wie die Mesylate, Tosylate, Brosylate und Benzolsulfonate. Besonders geeignet sind die Sulfate, Halogenide und Sulfonsäureester, von denen die Sulfate bevorzugt werden. Der Esterrest $R^1$ stellt gegebenenfalls Substituenten tragende Kohlenwasserstoffreste mit 1 bis 10 C-Atomen dar. Geeignete Reste und Substituenten sind in der DE-A-26 16 588 beschrieben.

Beispiele für Alkylierungsmittel, die für dieses Verfahren eingesetzt werden können, sind Dimethylsulfat, Diethylsulfat, Dihexylsulfat, Diallylsulfat, Dicrotylsulfat, Di(β-phenylethyl)sulfat, Di(β-phenylallyl)sulfat, Di(β-methoxyethyl)sulfat, Di(β-phenoxyethyl)sulfat, Di(methylthioethyl)sulfat oder Di(β-phenylthioethyl)sulfat, Benzylbromid, Allylbromid, Benzolsulfonsäuremethylester, Benzolsulfonsäureethylester, p-Toluolsulfonsäuremethylester, p-Toluolsulfonsäureethylester oder das o-, p-Gemisch der entsprechenden Toluolsulfonsäureester. Bevorzugt werden beispielsweise die leicht zugänglichen o-,p-Gemische des Toluolsulfonsäuremethylesters oder das Dimethylsulfat.

Als Alkoholate werden wahlweise dem Alkylierungsmittel entsprechende (für symmetrische Acetale) bzw. nicht entsprechende Alkoholate (für unsymmetrische Acetale) mit Resten $R^2$ verwendet, also beispielsweise bei Verwendung von Dimethylsulfat ein Ethylat wie Natriumethylat oder Kaliumethylat oder bei Verwendung von Allylbromid z.B. Natriummethylat. Ansonsten gelten bezüglich der Art der Reste $R^2$ die vorstehenden Angaben zu den Resten $R^1$ sinngemäß. Bevorzugt sind die Alkoholate des Natriums und Kaliums, die vorzugsweise in fester kristalliner Form oder als Aufschlämmung im entsprechenden org. Lösungsmittel eingesetzt werden.

Als Katalysatoren werden Harnstoffderivate der allgemeinen Formel III oder IV verwendet. Geeignete Beispiele für Derivate der Formel III sind Dimethylharnstoff, Diethylharnstoff, Tetramethylharnstoff, Tetraethylharnstoff, Tetrabutylharnstoff oder deren Gemische. Bevorzugt sind Dimethylharnstoff, Tetramethylharnstoff oder Tetrabutylharnstoff.

Geeignete Beispiele für Derivate der Formel IV sind N,N′-Propylenharnstoff, N-Methylpropylenharnstoff, N,N′-Dimethylpropylenharnstoff, N,N′-Ethylenharnstoff, N-Methylethylenharnstoff, N,N′-Dimethylethylenharnstoff sowie deren Gemische. Bevorzugt sind N-Methylpropylenharnstoff und N,N′-Dimethylpropylenharnstoff.

Es können aber auch Gemische der Harnstoffderivate der Formeln III und IV verwendet werden. Die Harnstoffderivate werden in Mengen von 0,5 bis 50 Gew.%, bevorzugt 5 bis 30 Gew.%, bezogen auf das jeweils eingesetzte Diketon verwendet.

Von den organischen Lösungsmitteln oder Lösungsmittelgemischen kommen ausschließlich unpolare Lösungsmittel in Betracht. Im Handbook of Chemistry and Physics (62nd Ed.), Seite C-700, sind Beispiele aufgeführt. Von diesen werden wiederum die aromatischen Lösungsmittel bevorzugt, die einen Siedepunkt im Bereich von 60 bis 140°C haben. Die Menge an Lösungsmittel sollte zweckmäßigerweise so bemessen sein, daß das Reaktionsgemisch nach Zusammengeben aller Reaktionskomponenten noch gut rührbar ist. Dies ist im allgemeinen dann der Fall, wenn das Lösungsmittel wenigstens etwa die Hälfte des Reaktionsgemisches ausmacht.

Die erfindungsgemäße Umsetzung der 1,2-Diketone mit dem Alkylierungsmittel und Alkoholat erfolgt im allgemeinen bei Temperaturen von -20°C bis 100°C, bevorzugt bei 20 bis 40°C. Die Höhe und Einhaltung der Innentemperatur ist für den Erfolg der Reaktion wichtig. Sie hängt von der Reaktivität des aus dem Diketon und dem Alkoholat gebildeten Anions ab. Bei Diketonen mittlerer Reaktivität, z.B. dem 4,4'-Dimethylbenzil sollte sie 35 bis 40°C nicht überschreiten. Bei Ketonen, die sehr langsam mit dem Alkoholat/Alkylierungsmittel reagieren, muß temperaturgesteuert zudosiert werden. Im allgemeinen ist die Reaktion jedoch innerhalb weniger Minuten abgeschlossen und in den meisten Fällen ist sie bereits beendet, sobald die Reaktionsteilnehmer zusammengebracht worden sind. In diesem Punkt unterscheidet sich das vorliegende Verfahren deutlich von der in den DE-A 26 16 382, DE-A 26 16 408 und DE-A 26 16 508 beschriebenen Methode mit langen Nachrührzeiten.

Theoretisch werden 1/n Mol Alkylierungsmittel der vorstehend erläuterten Formel $(R^1)_nX$ mit einem Mol 1,2-Diketon und 1/m Mol Alkoholat der ebenfalls vorstehend erläuterten Formel $(R^2O)_mM$ umgesetzt, wobei n und m der Zahl n bzw. m in der Formel des verwendeten Alkylierungsmittel bzw. Alkoholats entsprechen, um die unsymmetrischen 1,1-Acetale der aromatischen 1,2-Diketone zu erzeugen. Im allgemeinen wird es jedoch vorgezogen, einige Reaktionsteilnehmer in überschüssigen Mengen einzusetzen, um eine vollständige Umsetzung zu erreichen. So kann man pro Mol 1,2-Diketon 1/n bis 10/n Mol oder mehr, vorzugsweise 1/n bis 4/n Mol Alkylierungsmittel und 1/m bis 10/m Mol oder mehr, vorzugsweise 1/m bis 4/m Mol Alkoholat einsetzen.

Bei einer zweckmäßigen Ausführungsform der Erfindung wird das Verfahren so durchgeführt, daß das Alkoholat in ein Reaktionsgemisch, bestehend aus dem aromatischen 1,2-Diketon, dem Alkylierungsmittel, dem Katalysator und dem Lösungsmittel, eingetragen wird. Dabei kann das Alkoholat direkt als Festsubstanz oder auch in Form einer Suspension, z.B. in Toluol, Xylol oder o-Dichlorbenzol zugegeben werden.

Das Verfahren wird am zweckmäßigsten so ausgeführt, daß das pulverförmige, kristalline Alkoholat temperaturgesteuert kontinuierlich zudosiert wird. Der Zusatz von Wasser oder protischen Lösungsmitteln, wie z.B. Alkohol stört bzw. inhibiert die Alkylierung.

Nach Beendigung der Reaktion, was beispielsweise dünnschicht- oder gaschromatographisch sehr leicht feststellbar ist, werden eventuell verbliebene Reste an Dimethylsulfat durch Zusatz von Basen, wie Ethanolamin, Ammoniumhydroxid, wäßriger Natronlauge oder durch Hydrolyse von überschüssigem Natrium- oder Kaliumalkoholat zerstört. Dabei muß selbstverständlich darauf geachtet werden, daß das Reaktionsgemisch nicht in den sauren pH-Bereich gelangt, da sonst eine Hydrolyse des 1,1-Acetals zum entsprechenden Diketon erfolgen kann.

Die Aufarbeitung des Reaktionsgemisches und Isolierung des Reaktionsproduktes kann nach herkömmlichen Verfahren wie Ausfällen, Extraktion oder Destillation erfolgen. Eine mögliche Vefahrensweise besteht z.B. darin, nach dem Vernichten des überschüssigen Alkylierungsmittels das Toluol auszukreisen, eine Phasentrennung durchzuführen und das mit Wasser nicht mischbare Acetal durch Kristallisation oder Destillation zu reinigen. Voraussetzung für diese Arbeitsweise ist jedoch, daß das zur Umsetzung verwendete Lösungsmittel nicht mit Wasser mischbar ist und ein Azeotrop mit Wasser bildet. Das so isolierte Acetal fällt in analysenreiner Form an. Das Verfahren bietet den Vorteil, daß das in die Reaktion eingesetzte Lösungsmittel und der Katalysator aus den entsprechenden Phasen nach klassischen Methoden wieder weitgehend zurückgewonnen werden kann.

Beispiel 1

Herstellung von Benzildimethylketal

Methode A:

In 400 ml Toluol wurden bei Raumtemperatur 84 g Benzil, 15 g N,N'-Propylenharnstoff und 65 ml Dimethylsulfat gelöst. Zu der klaren gelben Lösung wurden dann 5 Mal 7,5 g Natriummethylat so zugegeben, daß

die Innentemperatur 30°C nicht überschritt. Nach beendeter Zugabe wurden 20 ml 20 gew.%ige Natronlauge zugetropft und das Reaktionsgemisch bei Normaldruck auf 60°C erhitzt. Nach 20 Minuten wurde ein Vakuum von 300 mbar angelegt und zunächst das Azeotrop 20 Gew.% Wasser/80 Gew.% Toluol und anschließend reines Toluol abdestilliert. Nach Zusatz von 400 ml 10 gew.%iger wäßriger Natronlauge wurde das restliche Toluol abdestilliert. Bei 70°C wurde anschließend eine Phasentrennung durchgeführt und die organische Phase aus Isopropanol/Wasser umkristallisiert.

Ausbeute: 96 g (94 %) farblose Nadeln vom Schmp. 63-65°C.

Methode B:

Das bei der Phasentrennung erhaltene rohe Benzildimethylketal wurde bei einem Druck von 10 mbar an einer kurzen Füllkörperkolonne fraktioniert. Nach einem geringen Vorlauf von Restwasser geht reines Benzildimethylketal als Öl mit einem Siedepunkt von 165°C/10 mbar über, das beim Stehen in farblosen Nadeln kristallisiert.

Ausbeute: 98 g (96 %)

Methode C:

Das bei der Phasentrennung erhaltene rohe Benzildimethylketal wurde als Schmelze langsam in ein Gemisch aus 720 ml Wasser und 80 ml Methanol eingerührt, wobei es in Form feiner farbloser Prismen ausfiel. Nach dem Absaugen und Waschen mit Wasser/Methanol = 20:1 wurde im Vakuum getrocknet.

Ausbeute: 95 g (93 %)

Beispiele 2 bis 15

Variation des Katalysators

In Abänderung von Beispiel 1, Methode A, wurde der Katalysator N,N'-Propylenharnstoff durch die in Tabelle 1 aufgeführten Katalysatoren gemäß Formel III ersetzt. Die folgenden Ausbeuten wurden erhalten:

Tabelle 1

| Beispiel Nr. | Katalysator | Ausbeute an isoliertem BDK [%] |
|---|---|---|
| 2* | - | 48 |
| 3* | Ethylenglykoldimethylether | 58 |
| 4* | Diethylenglykolmonomethylether | 4 |
| 5* | Diethylenglykolmonoethylether | 50 |
| 6* | Diethylenglykolmonobutylether | 8 |
| 7* | 1,2-Propandiol | 42 |
| 8* | Pluriol® E 1000 | 53 |
| 9* | Dimethylformamid | 72 |
| 10 | N,N'-Dimethylpropylenharnstoff | 97 |
| 11 | N-Methylpropylenharnstoff | 92 |
| 12 | N,N'-Ethylenharnstoff | 85 |
| 13 | N-Methyl-ethylenharnstoff | 82 |
| 14 | N,N'-Dimethylethylenharnstoff | 96 |
| 15 | Tetramethylharnstoff | 88 |

*) Zum Vergleich.

In den Beispielen 2 bis 8 wurden doppelte Reaktionszeiten gewählt. In den Beispielen 3 bis 6 trat nach

einer unterschiedlich langen Inhibierungsphase eine spontane, exotherme Reaktion ein, die bei 8 und 9 mit einer starken Gasentwicklung verbunden war.

Beispiel 16

Herstellung von Benzil-1-methyl-1-ethylacetal

In Analogie zu Beispiel 1, Methode A, wurde Benzil mit Dimethylsulfat und Natriumethylat in Gegenwart von N,N'-Dimethylpropylenharnstoff umgesetzt. Nach der Phasentrennung wurde reines Wertprodukt als gelbliches Öl erhalten, das nach einwöchigem Stehen allmählich kristallisierte.

Ausbeute: 79 %, Schmp. 53-54°C.

Beispiel 17 bis 19

Herstellung weiterer Dialkylacetale

In Anlehnung an die in Beispiel 1 beschriebene Methode wurden folgende Dialkylacetale synthetisiert:

Tabelle 2

| Beispiel | Dialkylacetal | Methode | Katalysator | Ausbeute |
|---|---|---|---|---|
| 17* | Phenanthrenchinondimethylacetal | A Essigester/ Hexan | N-Methylpropylenharnstoff | 65 |
| 18 | 2,2-Dimethoxyacenaphthylen-1-(2H)-on | A Hexan | Tetramethylharnstoff | 83 |
| 19* | 2-Ethoxy-2-methoxyacenaphthylen-1-(2H)-on | Isopropanol/ Wasser | N,N'-Dimethylpropylenharnstoff | 85 |

*) Zum Vergleich.

**Patentansprüche**

1. Verfahren zur Herstellung von symmetrischen und unsymmetrischen 1,1-Acetalen aromatischer 1,2-Diketone der allgemeinen Formel I

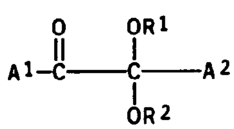

I

worin

$A^1$ und $A^2$ für gleiche, verschiedene oder miteinander verbundene, gegebenenfalls geradkettige oder verzweigte Alkyl- oder Alkoxy-Reste mit 1 bis 6 C-Atomen, Halogen- oder Phenylreste tragende, aromatische Reste stehen und

$R^1$ und $R^2$ für gleiche oder verschiedene, gegebenenfalls substituierte, geradkettige oder verzweigte Alkylreste mit 1 bis 10 C-Atomen stehen,

aus den entsprechenden aromatischen 1,2-Diketonen der allgemeinen Formel II

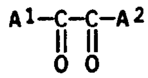

II,

dadurch gekennzeichnet, daß man die 1,2-Diketone in einem unpolaren aprotischen organischen Lösungsmittel mit einem Alkylierungsmittel

$(R^1)_nX$

und einem Alkoholat

$(R^2O)_mM$,

wobei n und m für ganze Zahlen von 1 bis 3 stehen,
X für den Rest einer ein- bis dreibasigen Säure und
M für ein Metall der 1. bis 3. Hauptgruppe des Periodensystems der Elemente steht,
in Gegenwart eines Harnstoffderivates der allgemeinen Formel III oder IV

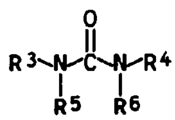

III

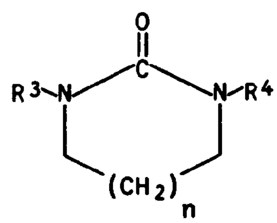

IV

oder eines Gemisches von III oder IV,
worin
n = 0 bis 3 und
$R^3$, $R^4$, $R^5$ und $R^6$ für gleiche oder verschiedene, geradkettige oder verzweigte Alkylreste mit 1 bis 6 C-Atomen oder Wasserstoff stehen, als Katalysator bei Temperaturen von -20 bis +100°C umsetzt.

2. Verfahren nach Anspruch 1, dadurch gekennzeichnet, daß man ein mit Wasser nicht mischbares, unpolares organisches Lösungsmittel verwendet.

3. Verfahren nach einem der Ansprüche 1 oder 2, dadurch gekennzeichnet, daß man Dimethylharnstoff und/oder Tetramethylharnstoff als Katalysatoren der allgemeinen Formel III verwendet.

4. Verfahren nach einem der Ansprüche 1 oder 2, dadurch gekennzeichnet, daß man Ethylenharnstoff und/oder Propylenharnstoff sowie deren mono- und/oder bis-(N-$C_1$-$C_6$-Alkyl)derivate als Katalysatoren der allgemeinen Formel IV verwendet.

## Claims

1. A process for the preparation of a symmetric or asymmetric 1,1-acetal of an aromatic 1,2-diketone, of the general formula I

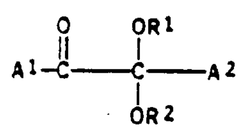

where $A^1$ and $A^2$ are straight-chain or branched alkyl or alkoxy radicals of 1 to 6 carbon atoms, which are identical, different or bonded to one another, or aromatic radicals carrying halogen or phenyl radicals and $R^1$ and $R^2$ are identical or different, unsubstituted or substituted, straight-chain or branched alkyl radicals of 1 to 10 carbon atoms, from the corresponding aromatic 1,2-diketone of the general formula II

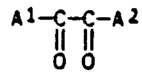

wherein the 1, 2 -diketone, in a nonpolar aprotic organic solvent, is reacted with an alkylating agent

$$(R^1)_nX$$

and an alcoholate

$$(R^2O)_mM,$$

where n and m are integers of from 1 to 3, X is a radical of a monobasic to tribasic acid and M is a metal of main groups 1 to 3 of the periodic table of the elements, in the presence of a urea derivative of the general formula III or IV

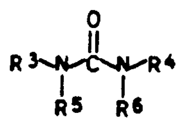

III

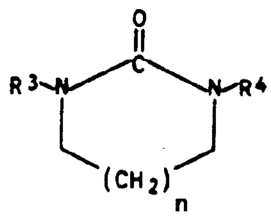

IV

or of a mixture of III or IV, where n is from 0 to 3 and $R^3$, $R^4$, $R^5$ and $R^6$ are identical or different, straight-chain or branched alkyl radicals of 1 to 6 carbon atoms or hydrogen, as a catalyst and from -20 to + 100°C.

2. A process as claimed in claim 1, wherein a water-immiscible, nonpolar organic solvent is used.

3. A process as claimed in either of claims 1 and 2, wherein dimethyl urea or tetraethylurea or a mixture thereof is used as the catalyst of the general formula III.

4. A process as claimed in either of claims 1 and 2, wherein ethyleneurea, propyleneurea, one of their mono- or bis-($N$-$C_1$-$C_6$-alkyl) derivatives or a mixture of these compounds is used as the catalyst of the general formula IV.

## Revendications

1. Procédé de préparation de 1,1-acétals, symétriques et asymétriques, de 1,2-dicétones aromatiques de la formule générale I

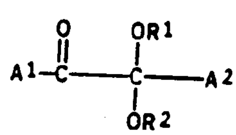

I

dans laquelle
les symboles $A^1$ et $A^2$ représentent des radicaux aromatiques, identiques, différents ou mutuellement reliés, qui portent éventuellement des atomes d'halogènes, ou des radicaux phényle, ou des radicaux alcoxy ou alkyle, comportant de 1 à 6 atomes de carbone, à chaîne droite ou à chaîne ramifiée et

les symboles $R^1$ et $R^2$ représentent des radicaux alkyle comportant de 1 à 10 atomes de carbone, à chaîne droite ou à chaîne ramifiée, éventuellement substitués, identiques ou différents, à partir des 1,2-dicétones aromatiques correspondantes de la formule générale II

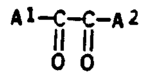 II,

caractérisé en ce que l'on fait réagir les 1,2-dicétones dans un solvant organique aprotique et apolaire, avec un agent d'alkylation

$$(R^1)_nX$$

et un alcoolate

$$(R^2O)_mM$$

où n et m représentent des nombres entiers qui varient de 1 à 3,
X représente le reste d'un acide mono à tribasique et
M représente un métal des premier à troisième groupes principaux du système périodique des éléments, en présence d'un dérivé d'urée de la formule générale III ou IV

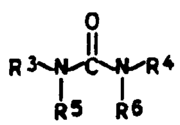 III

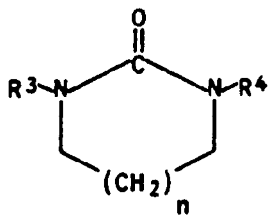 IV

ou d'un mélange de III et de IV,
où
n = 0 à 3 et
les symboles $R^3$, $R^4$, $R^5$ et $R^6$ représentent de l'hydrogène ou des radicaux alkyle qui comportent de 1 à 6 atomes de carbone, à chaîne droite ou à chaîne ramifiée, identiques ou différents, servant de catalyseur, à des températures de -20 à +100°C.

**2.** Procédé suivant la revendication 1, caractérisé en ce que l'on utilise un solvant organique, apolaire, non miscible à l'eau.

**3.** Procédé suivant l'une quelconque des revendications 1 et 2, caractérisé en ce que l'on utilise la diméthylurée et/ou la tétraméthylurée à titre de catalyseurs de la formule générale III.

**4.** Procédé suivant l'une quelconque des revendications 1 et 2, caractérisé en ce que l'on utilise de l'éthylèneurée et/ou de la propylèneurée, comme aussi leurs dérivés mono- et/ou bis-(N-alkyliques en $C_1$-$C_6$) à titre de catalyseurs de la formule IV.